(19) Europäisches Patentamt
European Patent Office
Office européen des brevets

(11) EP 0 541 445 B1

(12) # FASCICULE DE BREVET EUROPEEN

(45) Date de publication et mention de la délivrance du brevet:
**21.02.1996 Bulletin 1996/08**

(51) Int Cl.[6]: **C07D 307/93**, C07C 62/02

(21) Numéro de dépôt: **92402994.5**

(22) Date de dépôt: **05.11.1992**

(54) **Nouveau procédé de préparation de la lactone de l'acide 1R,cis 2,2-diméthyl 3-formyl cyclopropane-1-carboxylique et intermédiaires**

Verfahren zur Herstellung von 1R, cis-2,2-Dimethyl-3-Formyl-Cyclopropan-1-Carbonsäurelacton und Zwischenprodukten

Procedure for preparing 1R, cis-2,2-dimethyl-3-formyl cyclopropane-1-carboxylic acid lactone and intermediates

(84) Etats contractants désignés:
**AT BE CH DE DK ES FR GB GR IE IT LI LU NL PT SE**

(30) Priorité: **08.11.1991 FR 9113775**

(43) Date de publication de la demande:
**12.05.1993 Bulletin 1993/19**

(73) Titulaire: **ROUSSEL UCLAF**
**F-93230 Romainville (FR)**

(72) Inventeurs:
- **Brion, Francis**
  **F-93220 Gagny (FR)**
- **Colladant, Colette**
  **F-93110 Rosny Sur Bois (FR)**
- **Lagouardat, Jacques**
  **F-93160 Noisy Le Grand (FR)**
- **Scholl, Jacques**
  **F-93230 Romainville (FR)**

(74) Mandataire: **Vieillefosse, Jean-Claude et al**
**F-93235 Romainville Cédex (FR)**

(56) Documents cités:
**CH-A- 527 152** **GB-A- 2 002 377**

- **HOUBEN-WEYL 'Methoden der Organischen Chemie, ed. 4, vol. 4/1A (Oxidation, Teil I), "Anorganische Halogen-Verbindungen als Oxidationsmittel", pages 435 - 640' 17 Décembre 1981 , GEORG THIEME VERLAG , STUTTGART, DE**
- **HOUBEN-WEYL 'Methoden der Organischen Chemie, ed. 4, vol. 4/1B (Oxidation, Teil II), "Wismut- und Vanadin-Verbindungen als Oxidationsmittel", pages 415 - 424' 11 Décembre 1975 , GEORG THIEME VERLAG , STUTTGART, DE**

EP 0 541 445 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

Printed by Jouve, 75001 PARIS (FR)

## Description

La présente invention a pour objet un nouveau procédé de préparation de la lactone de l'acide 1R,cis 2,2-diméthyl 3-formyl cyclopropane-1-carboxylique et des intermédiaires. Un procédé de préparation de composé de formule (I) au départ d'un composé de formule (II) a déjà été décrit dans la demande de brevet anglais 2 002 377. Ce procédé consiste en une cyclodéshydratation conduisant à une énol lactone intermédiaire qui est traitée par l'ozone puis clivée. L'invention a ainsi pour objet un procédé de préparation du composé de formule (I) :

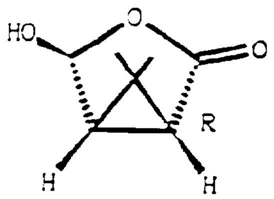

(I)

caractérisé en ce que l'on traite le composé de formule (II) :

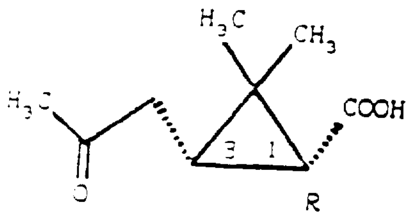

(II)

de configuration 1R,cis, par un hypohalogénite alcalin, alcalino-terreux ou de magnésium, pour obtenir le composé de formule (III) :

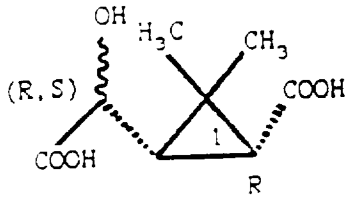

(III)

de configuration 1R,cis, pouvant également exister sous la forme cyclisée lactonique (III') :

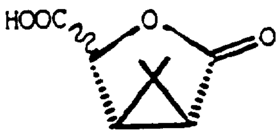

(III')

l'une ou l'autre forme se présentant en tant que mélange de diastéréoisomères et sous forme de sel alcalin, alcalino-terreux ou de magnésium, à partir duquel l'on isole, le cas échéant, l'acide, puis le cas échéant, les diastéréoisomères, puis traite le composé de formule (III) ou (III′) sous la forme d'un mélange de diastéréoisomères ou de diastéréoisomères séparés, ou son sel, par un agent oxydant, pour obtenir le composé de formule (I).

L'hypohalogénite alcalin, alcalino-terreux ou de magnésium peut être un hypochlorite, un hypobromite ou un hypoiodite de sodium, de potassium, de lithium, de calcium ou de magnésium, et de préférence, l'hypochlorite de sodium.

Dans des conditions préférées de mise en oeuvre du procédé de l'invention, on utilise 4 équivalents au moins d'hypohalogénite alcalin ou alcalino-terreux et notamment d'hypochlorite de sodium et l'on opère à une température pouvant aller de - 10 à + 20°C et, plus particulièrement, de - 5 à + 5°C, en phase aqueuse.

En outre, il peut être avantageux d'opérer en présence d'un agent basique, lequel peut notamment être choisi dans le groupe constitué par les hydroxydes alcalins et alcalino-terreux et, plus particulièrement, les hydroxydes de sodium, de potassium et de calcium.

La séparation éventuelle des diastéréoisomères de formule (III) ou (III′) peut être effectuée par des moyens classiques, notamment la chromatographie ou la cristallisation. Des exemples sont décrits ci-après dans la partie expérimentale.

L'isolement de l'acide de formule (III) ou (III′) peut être effectué par acidification du milieu réactionnel, de préférence après neutralisation du pouvoir oxydant par un agent réducteur, par exemple le thiosulfate de sodium, puis extraction par des moyens classiques. L'isolement du sel est possible en amenant à sec le milieu réactionnel de préférence après

neutralisation du pouvoir oxydant.

L'agent oxydant avec lequel l'on traite le composé de formule (III) ou (III') peut notamment être choisi dans le groupe constitué par les acides hypohalogéneux, les hypohalogénites alcalins, alcalino-terreux et de magnésium, le permanganate de potassium, l'acide chromique, l'acide périodique et les bismuthates alcalins. Il peut encore être, par exemple le dioxyde de manganèse ou un perborate. Un acide hypohalogéneux et notamment l'acide hypochloreux est plus particulièrement préféré.

Dans des conditions préférées de mise en oeuvre du procédé, l'acide hypohalogéneux utilisé comme oxydant est obtenu in situ à partir d'un hypohalogénite alcalin, alcalino-terreux ou de magnésium placé en milieu acide. L'acide hypochloreux est ainsi obtenu in situ à partir d'hypochlorite de sodium.

L'acide utilisé pour libérer l'acide hypochloreux est de préférence choisi dans le groupe constitué par les acides alcanoïques inférieurs, tels que l'acide acétique ou propionique, ainsi que des solutions de phosphates, borates et acétates, de pH approprié.

L'invention a tout particulièrement pour objet un procédé tel que défini précédemment, caractérisé en ce que l'on opère sans isoler intermédiairement le composé de formule (III) ou (III').

Dans ce cas, les conditions opératoires et notamment l'agent oxydant utilisé peuvent être les mêmes que celles qui ont été définies plus haut. Toutefois, l'utilisation d'un acide hypohalogéneux ou d'un hypohalogénite alcalin ou alcalino-terreux est particulièrement préférée. On opère alors avantageusement en utilisant au départ une quantité supérieure à 4 équivalents d'hypohalogénite, notamment d'hypochlorite de sodium.

Il résulte de ce qui précède que le procédé consistant à mettre le produit de formule (II) en présence du seul hypohalogénite, agissant donc également comme agent oxydant, entre dans le cadre de l'invention. Un tel procédé est illustré dans la partie expérimentale.

Le composé de formule (II) utilisé au départ du procédé de l'invention est connu par exemple par la publication Agr. Biol. Chem. Vol 29 N° 8 p. 784 (1965).

On peut préciser à titre purement indicatif et non limitatif de l'invention que l'action de l'hypohalogénite en milieu aqueux sur le composé de formule (II) entraîne la formation in situ de composés mono et polyhalogénés sur la chaîne en position 3, ceux-ci étant des intermédiaires dans la formation du composé de formule (III) ou (III'). Ces composés intermédiaires constituent l'un des objets de la demande de brevet n° 91 13776 déposée le même jour que la présente demande par la demanderesse et également intitulée : "Nouveau procédé de préparation de la lactone de l'acide 1R,cis 2,2-diméthyl 3-formyl cyclopropane-1-carboxylique et intermédiaires et halogénés".

L'invention a enfin pour objet, à titre de produits industriels nouveaux et notamment à titre d'intermédiaires nécessaires à la mise en oeuvre du procédé de l'invention, les composés de formule (III) et (III') tels que définis précédemment, ainsi que leurs sels alcalins, alcalino-terreux et de magnésium, sous forme de mélanges de diastéréoisomères ou de diastéréoisomères séparés.

Le composé de formule (I) est décrit dans le brevet français 1580474. Il s'agit d'un important intermédiaire dans la synthèse d'esters bien connus possédant notamment une activité insecticide, ainsi qu'il ressort du brevet français 2 396 006. Les exemples suivants illustrent l'invention sans toutefois la limiter.

**Exemple 1 : Lactone de l'acide 1R,cis 2,2-diméthyl 3-formyl cyclopropane-1-carboxylique ou (1S-(1alpha,2béta, 5alpha))-6,6-diméthyl-4-oxo-3-oxabicyclo [3.1.0] hexan-2-ol.**

Stade A :

Acide 1R,cis 2,2-diméthyl 3-(hydroxy carboxy méthyl) cyclopropane-1-carboxylique.

A une solution de 1 g d'acide 1R,cis 2,2-diméthyl 3-(2-oxo propyl) cyclopropane-1-carboxylique dans 10 $cm^3$ d'eau est ajoutée 13,2 $cm^3$ d'une solution aqueuse d'hypochlorite de sodium à 31,255 g de NaClO pour 100 $cm^3$ de solution (47° chlorométriques). On maintient sous agitation à température ambiante pendant 2 heures, puis rajoute 2,2 $cm^3$ de la solution d'hypochlorite de sodium et poursuit l'agitation pendant 30 minutes. On détruit le pouvoir oxydant du milieu par addition d'une solution aqueuse de thiosulfate de sodium puis l'acidifie à pH 2,5 par addition d'acide chlorhydrique concentré et le sature par du sulfate d'ammonium. On extrait au chlorure de méthylène et à l'acétate d'éthyle, sèche la phase organique et l'amène à sec. On obtient 0,6 g d'acide attendu que l'on peut recristalliser dans l'éther isopropylique.

Stade B :

Lactone de l'acide 1R,cis 2,2-diméthyl 3-formyl cyclopropane-l-carboxylique. On mélange 4 g d'acide obtenu comme décrit au stade A, 54 $cm^3$ d'eau, 26 $cm^3$ d'acide acétique et 40 $cm^3$ de chlorure de méthylène. On introduit sous agitation en 30 minutes environ, à température ambiante, 16 $cm^3$ d'une solution aqueuse d'hypochlorite de sodium à 31,92 g de NaClO pour 100 $cm^3$ de solution (48° chlorométriques). On poursuit l'agitation pendant 1 heure, puis détruit le pouvoir

oxydant du milieu par addition d'une solution aqueuse de thiosulfate de sodium. On acidifie à pH 2,5 par addition d'acide chlorhydrique concentré et sature par addition de sulfate d'ammonium. On extrait au chlorure de méthylène et obtient après séchage et évaporation du solvant 2,68 g de lactone brute attendue. on empâte 2 g de produit dans un mélange eau-toluène, essore et sèche. On obtient 1,6 g de produit attendu. F=113°C. $alpha_D^{20}$ = - 110,5° (c=1 % DMF).

**Exemple 2 : Lactone de l'acide 1R,cis 2,2-diméthyl 3-formyl cyclopropane-1-carboxylique ou (1S-(1alpha,2béta, 5alpha))-6,6-diméthyl-4-oxo-3-oxabicyclo [3.1.0] hexan-2-ol.**

On mélange à température ambiante 20 mg d'acide obtenu au stade A de l'exemple 1 et 1 $cm^3$ d'eau, puis ajoute 15 mg de permanganate de potassium et maintient sous agitation à température ambiante pendant 20 heures. On traite l'essai comme au stade B de l'exemple 1, acidification exceptée et obtient le produit attendu avec un rendement de 50 % environ.

**Exemple 3 : Lactone de l'acide 1R,cis 2,2-diméthyl 3-formyl cyclopropane-1-carboxylique ou (1S-(1alpha,2béta, 5alpha))-6,6-diméthyl-4-oxo-3-oxabicyclo [3.1.0] hexan-2-ol.**

On mélange à température ambiante 20 mg d'acide obtenu au stade A de l'exemple 1 et 1 $cm^3$ d'eau, puis ajoute du mélange sulfochromique en léger excès. On maintient sous agitation à température ambiante pendant 20 heures. On traite l'essai comme au stade B de l'exemple 1, acidification exceptée et obtient le produit attendu avec un rendement de 50 % environ.

**Exemple 4 : Lactone de l'acide 1R,cis 2,2-diméthyl 3-formyl cyclopropane-1-carboxylique ou (1S-(1alpha,2béta, 5alpha))-6,6-diméthyl-4-oxo-3-oxabicyclo [3.1.0] hexan-2-ol.**

On mélange à température ambiante 20 mg d'acide obtenu au stade A de l'exemple 1 et 1 $cm^3$ d'eau, puis ajoute 20 mg d'acide périodique et maintient sous agitation à température ambiante pendant 20 heures. On traite l'essai comme au stade B de l'exemple 1, acidification exceptée et obtient le produit attendu avec un rendement de 10 % environ.

**Exemple 5 : Lactone de l'acide 1R,cis 2,2-diméthyl 3-formyl cyclopropane-1-carboxylique ou (1S-(1alpha,2béta, 5alpha))-6,6-diméthyl-4-oxo-3-oxabicyclo [3.1.0] hexan-2-ol.**

On mélange à température ambiante 20 mg d'acide obtenu au stade A de l'exemple 1 et 1 $cm^3$ d'eau, puis ajoute un excès de perborate de sodium et maintient sous agitation à température ambiante pendant 20 heures. On traite l'essai comme au stade B de l'exemple 1, acidification exceptée et obtient le produit attendu avec un rendement de 10 % environ.

**Exemple 6 : Lactone de l'acide 1R,cis 2,2-diméthyl 3-formyl cyclopropane-1-carboxylique ou (1S-(1alpha,2béta, 5alpha))-6,6-diméthyl-4-oxo-3-oxabicyclo [3.1.0] hexan-2-ol.**

On mélange 20 mg d'acide obtenu au stade A de l'exemple 1 et 2 $cm^3$ de chlorure de méthylène. On y ajoute 20 mg de dioxyde de manganèse préalablement activé par séchage (16 heures à 110°C). On porte au reflux sous agitation pendant 5 heures puis refroidit. On évalue par chromatographie sur couche mince le rendement en produit attendu à environ 10 %.

**Exemple 7 : Lactone de l'acide 1R,cis 2,2-diméthyl 3-formyl cyclopropane-1-carboxylique ou (1S-(1alpha,2béta, 5alpha))-6,6-diméthyl-4-oxo-3-oxabicyclo [3.1.0] hexan-2-ol.**

On mélange 100 mg d'acide obtenu au stade A de l'exemple 1 avec 2 $cm^3$ d'eau et 1 $cm^3$ d'acide acétique. On y ajoute à température ambiante, 330 mg de bismuthate de sodium et maintient sous agitation pendant 24 heures, après quoi l'on identifie et dose par HPLC le produit attendu, dans le milieu réactionnel. On obtient ainsi une quantité supérieure à 80 % de produit attendu.

**Exemple 8 : Acide 1R,cis 2,2-diméthyl 3-(hydroxy carboxy méthyl) cyclopropane-1-carboxylique.**

On mélange à 20°C, 2,2 g d'acide 1R,cis 2,2-diméthyl 3-(2-oxo propyl) cyclopropane-1-carboxylique et 4 $cm^3$ d'eau puis ajoute à 0, + 5°C 11,7 $cm^3$ de soude 2N puis une solution de 9,7 g d'hypochlorite de calcium titrant 65 %, dans 30 $cm^3$ d'eau. On maintient sous agitation pendant 1 heure à 0, + 2°C et ajoute une solution aqueuse de thiosulfate de

sodium à 20 % en quantité suffisante pour détruire le pouvoir oxydant du milieu. On ajoute ensuite de l'acide chlorhydrique concentré jusqu'à pH 1 à 2, 15 g de sulfate d'ammonium puis maintient sous agitation pendant 15 minutes, filtre et extrait le filtrat à l'acétate d'éthyle. On sèche et évapore le solvant. On obtient 2,5 g d'acide attendu sous la forme d'un produit semi-cristallisé, en mélange avec sa forme lactonique.

**Exemple 9 : Acide 1R,cis 2,2-diméthyl 3-(hydroxy carboxy méthyl) cyclopropane-1-carboxylique.**

On opère de manière analogue à celle décrite à l'exemple 8, en utilisant 17,15 g d'hypochlorite de lithium au lieu de la solution d'hypochlorite de calcium. On obtient 2,4 g de produit attendu semi-cristallisé, constitué par un mélange dudit produit avec sa forme lactonique.

**Exemple 10 : Acide 1R,cis 2,2-diméthyl 3-(hydroxy carboxy méthyl) cyclopropane-1-carboxylique.**

On opère de manière analogue à celle décrite à l'exemple 8, en utilisant 61 g d'une solution aqueuse d'hypobromite de sodium 2,2 M au lieu de la solution d'hypochlorite de calcium. On obtient 3,76 g de produit attendu brut, sous forme d'une huile constitué par un mélange dudit produit avec sa forme lactonique.

**Exemple 11 : Lactone de l'acide 1R,cis 2,2-diméthyl 3-formyl cyclopropane-1-carboxylique.**

On mélange 0,8 g d'acide obtenu comme décrit au stade A de l'exemple 1, 16 $cm^3$ d'eau et 4,2 $cm^3$ de soude 2N. On ajoute à température ambiante 2,4 $cm^3$ d'une solution aqueuse d'hypochlorite chlorite de sodium puis 2 gouttes d'acide acétique et maintient sous agitation pendant 1 heure. On ajoute ensuite une solution aqueuse de thiosulfate de sodium jusqu'à disparition du pouvoir oxydant, puis de l'acide chlorhydrique concentré jusqu'à pH 2,5 environ et enfin 10 g de sulfate d'ammonium. On extrait au chlorure de méthylène, sèche la phase organique et évapore à sec. On cristallise le résidu dans du toluène et obtient 0,2 g de produit attendu. F=114,5°C. $alpha_D^{20}$ = - 101° (c=1 % DMF).

**Exemple 12 :Lactone de l'acide 1R,cis 2,2-diméthyl 3-formyl cyclopropane-1-carboxylique.**

On mélange 0,5 g d'acide 1R,cis 2,2-diméthyl 3-(2-oxo propyl) cyclopropane-1-carboxylique, 6,5 $cm^3$ d'eau et 15,6 $cm^3$ d'une solution aqueuse d'hypochlorite de sodium à 31,255 g de NaC10 pour 100 $cm^3$ de solution (47° chlorométrique environ). On chauffe à 30°C pendant 2 heures, rajoute 7 $cm^3$ de la solution d'hypochlorite et poursuit l'agitation à 50°C pendant 3 heures. On refroidit à 5°C, ajoute du thiosulfate de sodium jusqu'à disparition du pouvoir oxydant, acidifie à pH 1 par de l'acide chlorhydrique concentré et extrait au chlorure de méthylène.

- On sèche la phase organique et évapore à sec.
- On obtient 0,215 g de produit attendu brut, que l'on peut purifier par cristallisation dans le toluène.

On obtient ainsi des cristaux identiques à ceux obtenus à l'exemple précédent.

**Exemple 13 : Lactone de l'acide 1R,cis 2,2-diméthyl 3-formyl cyclopropane-1-carboxylique.**

On mélange à température ambiante 30 g d'acide 1R,cis 2,2-diméthyl 3-(2-oxo propyl) cyclopropane-1-carboxylique, 60 $cm^3$ d'eau et ajoute 119 $cm^3$ de soude 2N. On ajoute à la solution obtenue, à 0, + 2°C, 433 $cm^3$ d'une solution aqueuse d'hypochlorite de sodium à 33,915 g de NaC10 pour 100 $cm^3$ de solution (51° chlorométrique). On maintient sous agitation pendant 1 h 30 puis mélange avec une solution de 329 $cm^3$ d'acide acétique et 90 $cm^3$ d'eau et 30 $cm^3$ de dichloroéthane. On agite à température ambiante puis traite par une solution de thiosulfate de sodium jusqu'à fin du pouvoir oxydant et amène à pH 3 par addition d'acide chlorhydrique concentré. On extrait au chlorure de méthylène, lave à l'eau et sèche. On obtient 27 g de produit attendu cristallisé brut que l'on peut purifier comme indiqué à l'exemple 11 ou 12.

**Exemple 14 : Lactone de l'acide 1R,cis 2,2-diméthyl 3-formyl cyclopropane-1-carboxylique.**

On opère comme indiqué à l'exemple 13, au départ de 3 g d'acide 1R,cis 2,2-diméthyl 3-(2-oxo propyl) cyclopropane-1-carboxylique, en utilisant 1,6 équivalents de soude 2N et 6 équivalents d'hypochlorite de sodium. On mélange la solution avec une solution de 30 $cm^3$ d'eau et 55 g de phosphate monosodique dihydraté et 30 $cm^3$ de dichloréthane. On procède ensuite comme indiqué à l'exemple 13 et obtient le produit attendu avec un rendement de 64 %.

**Exemple 15 : Isolement des diastéréoisomères de l'acide 1R,cis 2,2-diméthyl 3-(hydroxycarboxyméthyl) cyclopropane-1-carboxylique et de la lactone correspondante ou acide (1S-(1alpha, 2béta, 5alpha)) -6,6-diméthyl-4-oxo-3-oxabicyclo [3.1.0] hexane-2-carboxylique.**

On utilise au départ un mélange de diastéréoisomères obtenu selon l'un ou l'autre des exemples correspondants ci-dessus, soit 30 g que l'on traite pendant 1 h à température ambiante par 2 équivalents de soude 2N. On amène ensuite à pH 1 et extrait à l'acétate d'éthyle. On évapore à sec la phase organique et cristallise le résidu constitué d'un mélange d'acides, dans le chlorure de méthylène à 0°C. On reprend les 13 g de produit ainsi obtenu dans 10 volumes d'acide chlorhydrique N et porte à 50°C pendant 1 h. On extrait le mélange de lactones ainsi obtenu à l'acétate d'éthyle, évapore à sec et cristallise le résidu dans 4 volumes d'éther éthylique à 0°C. On obtient 1,8 g d'un des diastéréoisomères de la lactone. F=177°C.

$alpha_D^{20}$ = -100° (c=1 % DMF).

Spectre RMN ($CDCl_3$ 250 MHz)

1,20(s) et 1,26(s) : $CH_3$ gem ; 2,09(d,J=6) et 2,38(m) :

$H_1$ et $H_3$/cyclopropyles cis ; 5,12(d,J=6) : -CH-O.

On reprend 1,2 g de cet énantiomère dans 10 volumes de soude 2N et maintient sous agitation pendant 2 h à température ambiante. On ramène ensuite à pH 1 par addition d'acide chlorhydrique pur, à 0°C, extrait à l'acétate d'éthyle, évapore à sec et cristallise le résidu dans un mélange acétate d'éthyle/chlorure de méthylène. On obtient 0,8 g d'un des diastéréoisomères de l'acide. F=176°C.

$alpha_D^{20}$ = +9° (c=1 % DMF).

Spectre RMN (DMSO 250 MHz)

1,13(s) et 1,25(s) : $CH_3$ gem ; 1,32(m) et 1,51(d,J=8,5) :

$H_3$ et $H_1$ (cyclopropyles cis) ; 4,39(d,J=10,5) : -CHOH ;

5,24 et 12,08 : autres absorptions mobiles.

On peut obtenir l'autre diastéréoisomère de l'acide, en cristallisant le mélange d'acides dans 10 volumes de chlorure de méthylène à 0°C et en recristallisant le produit obtenu dans un mélange acétate d'éthyle-chlorure de méthylène (5/15). F=144°C. $alpha_D^{20}$= - 82,3° (c=1 % DMF).

Spectre RMN (DMSO 250 MHz)

1,09(s) et 1,24(s) : $CH_3$ gem ; 1,31(m) et 1,45(d,J=8,5) :

$H_3$ et $H_1$/cyclopropyles cis ; 4,29(d,J=10,5) : CHOH(R ou S).

La lactone correspondante peut être obtenue par chauffage de l'acide obtenu ci-dessus pendant 2 h à 50°C dans 10 volumes d'acide chlorhydrique N, puis, après refroidissement, par extraction à la méthyl éthyl cétone et évaporation à sec. F=100°C. $alpha_D^{20}$= -63,7° (c=1 % DMF).

Spectre RMN ($CDCl_3$ 250 MHz)

1,23(s) : $CH_3$ gem ; 2,08(d,J=6) et 2,31(d,J=6) : $H_1$ et $H_3$/cyclopropyles cis ; 4,71(s) : -CHO R ou S ; 8,36(s) : OH-.

## Revendications

1. Procédé de préparation du composé de formule (I) :

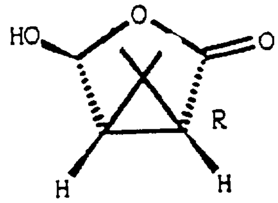

(I)

caractérisé en ce que l'on traite le composé de formule (II) :

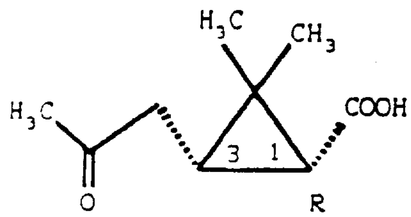

(II)

de configuration 1R,cis, par un hypohalogénite alcalin, alcalino-terreux, ou de magnésium pour obtenir le composé de formule (III) :

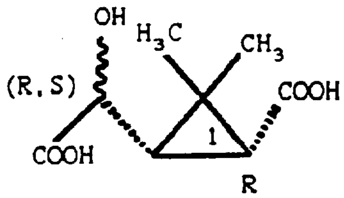

(III)

de configuration 1R,cis, pouvant également exister sous la forme cyclisée lactonique (III′) :

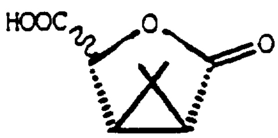

(III')

l'une ou l'autre forme se présentant en tant que mélange de diastéréoisomères, et sous forme de sel alcalin, alcalino-terreux ou de magnésium, à partir duquel l'on isole, le cas échéant, l'acide, puis, le cas échéant, les diastéréoisomères, puis traite le composé de formule (III) ou (III′), sous la forme d'un mélange de diastéréoisomères ou de diastéréoisomères séparés, ou son sel, par un agent oxydant, pour obtenir le composé de formule (I).

**2.** Procédé selon la revendication 1, caractérisé en ce que l'hypohalogénite est un hypochlorite, hypobromite ou hypoiodite de sodium, potassium, lithium, calcium ou magnésium et notamment l'hypochlorite de sodium.

**3.** Procédé selon la revendication 1 ou 2, caractérisé en ce que l'on utilise 4 équivalents au moins d'hypohalogénite.

**4.** Procédé selon l'une quelconque des revendications 1 à 3, caractérisé en ce que l'on opère à une température comprise entre - 5 et + 5°C, en phase aqueuse.

**5.** Procédé selon l'une quelconque des revendications 1 à 4, caractérisé en ce que l'on opère en présence d'un agent basique.

**6.** Procédé selon la revendication 5, caractérisé en ce que l'agent basique est choisi dans le groupe constitué par les hydroxydes alcalins et alcalino-terreux et notamment dans le groupe constitué par les hydroxydes de sodium, potassium et calcium.

**7.** Procédé selon la revendication 1, caractérisé en ce que l'agent oxydant est choisi dans le groupe constitué par les acides hypohalogéneux, les hypohalogénites alcalins, alcalino-terreux et de magnésium, le permanganate de potassium, l'acide chromique, l'acide périodique et les bismuthates alcalins et notamment un acide hypohalogéneux.

**8.** Procédé selon la revendication 7, caractérisé en ce que l'acide hypohalogéneux est obtenu in situ à partir d'un hypohalogénite alcalin, alcalino-terreux ou de magnésium placé en milieu acide.

**9.** Procédé selon la revendication 7, caractérisé en ce que l'agent oxydant est l'acide hypochloreux obtenu in situ à partir d'hypochlorite de sodium placé en milieu acide.

**10.** Procédé selon la revendication 8 ou 9, caractérisé en ce que l'acide est choisi dans le groupe constitué par les acides alcanoïques inférieurs, notamment les acides acétiques et propionique, et des solutions de phosphates,

borates et acétates.

**11.** Procédé selon la revendication 1, caractérisé en ce que l'on opère sans isoler intermédiairement le composé de formule (III) ou (III').

**12.** Procédé selon la revendication 11, caractérisé en ce que l'agent oxydant est tel que défini ou obtenu à l'une quelconque des revendications 7 à 10.

**13.** Procédé selon la revendication 11 ou 12, caractérisé en ce que l'on utilise au départ une quantité supérieure à 4 équivalents d'un hypohalogénite.

**14.** Procédé selon la revendication 13, caractérisé en ce que l'on utilise au départ une quantité supérieure à 4 équivalents d'hypochlorite de sodium.

**15.** Les composés de formule (III) et (III') telles que définies à la revendication 1, ainsi que leurs sels alcalins, alcalino-terreux ou de magnésium sous forme de mélanges de diastéréoisomères ou de diastéréoisomères séparés.

## Patentansprüche

**1.** Verfahren zur Herstellung der Verbindung der Formel (I)

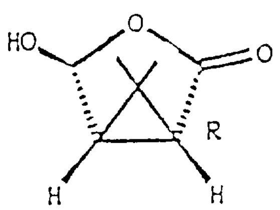

(I)

dadurch gekennzeichnet, daß man die Verbindung der Formel (II)

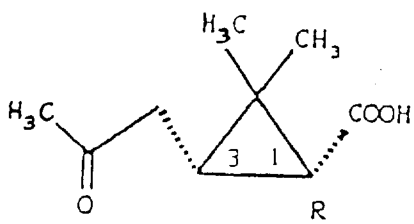

(II)

der Konfiguration 1R,cis mit einem Alkali-, Erdalkali- oder Magnesiumhypohalogenit behandelt, um die Verbindung der Formel (III)

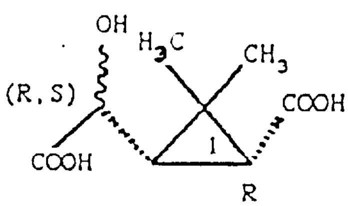

(III)

der Konfiguration 1R,cis zu erhalten, die auch in der cyclisierten Lactonform (III')

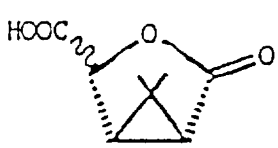

(III')

vorliegen kann, wobei die eine oder die andere Form als Gemisch der Diastereoisomeren und in Form des Alkali-, Erdalkali- oder Magnesiumsalzes vorliegt, von dem ausgehend man gegebenenfalls die Säure isoliert und dann gegebenenfalls die Diastereoisomeren, dann die Verbindung der Formel (III) oder (III') in Form eines Gemisches

der Diastereoisomeren oder die getrennten Diastereoisomeren oder sein Salz mit einem Oxidationsmittel behandelt, um die Verbindung der Formel (I) zu erhalten.

2. Verfahren gemäß Anspruch 1, dadurch gekennzeichnet, daß das Hypohalogenit ein Natrium-, Kalium-, Lithium-, Calciumoder Magnesiumhypochlorit, -hypobromit oder -hypojodit, und besonders das Natriumhypochlorit ist.

3. Verfahren gemäß Anspruch 1 oder 2, dadurch gekennzeichnet, daß man mindestens 4 Äquivalente Hypohalogenit verwendet.

4. Verfahren gemäß einem der Ansprüche 1 bis 3, dadurch gekennzeichnet, daß man bei einer Temperatur zwischen -5 °C und +5°C in wäßriger Phase arbeitet.

5. Verfahren gemäß einem der Ansprüche 1 bis 4, dadurch gekennzeichnet, daß man in Gegenwart eines basischen Mittels arbeitet.

6. Verfahren gemäß Anspruch 5, dadurch gekennzeichnet, daß das basische Mittel ausgewählt ist aus der Gruppe bestehend aus den Alkali- und Erdalkalihydroxiden und besonders aus der Gruppe bestehend aus den Natrium-, Kalium- und Calciumhydroxiden.

7. Verfahren gemäß Anspruch 1, dadurch gekennzeichnet, daß das oxidierende Mittel ausgewählt ist aus der Gruppe bestehend aus den hypohalogenigen Säuren, den Alkali-, Erdalkaliund Magnesiumhypohalogeniten, Kaliumpermanganat, Chromsäure, Perjodsäure und Alkaliwismutate, und besonders hypohalogenige Säure.

8. Verfahren gemäß Anspruch 7, dadurch gekennzeichnet, daß die hypohalogenige Säure in situ aus einem Alkali-, Erdalkalioder Magnesiumhypohalogenit in saurem Milieu in situ erhalten wird.

9. Verfahren gemäß Anspruch 7, dadurch gekennzeichnet, daß das Oxidationsmittel die hypochlorige Säure ist, die in situ ausgehend von Natriumhypochlorit, das in saurem Milieu vorliegt, erhalten wurde.

10. Verfahren gemäß Anspruch 8 oder 9, dadurch gekennzeichnet, daß die Säure ausgewählt ist aus der Gruppe bestehend aus den niederen Alkansäuren, besonders Essigsäure und Propionsäure und Lösungen von Phosphaten, Boraten und Acetaten.

11. Verfahren gemäß Anspruch 1, dadurch gekennzeichnet, daß man ohne die zwischenzeitliche Verbindung der Formel (III) oder (III') zu isolieren arbeitet.

12. Verfahren gemäß Anspruch 11, dadurch gekennzeichnet, daß das Oxidationsmittel ein solches ist, wie es in einem der Ansprüche 7 bis 10 definiert oder erhalten wurde.

13. Verfahren gemäß Anspruch 11 oder 12, dadurch gekennzeichnet, daß man zu Beginn eine Menge über 4 Äquivalente eines Hypohalogenits verwendet.

14. Verfahren gemäß Anspruch 13, dadurch gekennzeichnet, daß man zu Beginn eine Menge über 4 Äquivalente Natriumhypochlorit verwendet.

15. Die Verbindungen der Formel (III) und (III'), wie sie in Anspruch 1 definiert sind sowie ihre Alkali-, Erdalkali- oder Magnesiumsalze in Form von Gemischen der Diastereoisomeren oder getrennten Diastereoisomeren.

## Claims

1. Preparation process for the compound of formula (I):

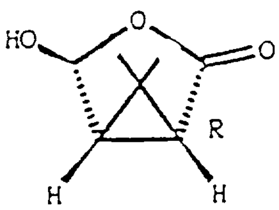

(I)

characterized in that the compound of formula (II):

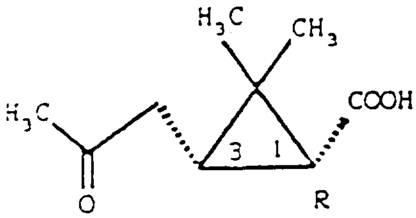

(II)

of 1R,cis configuration is treated with an alkaline, alkaline-earth or magnesium hypohalogenite, in order to obtain the compound of formula (III):

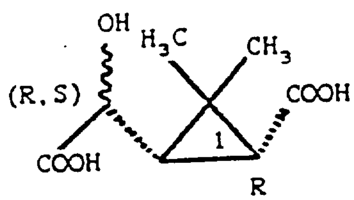

(III)

of 1R,cis configuration, also being able to exist in the lactonic cyclized form (III'):

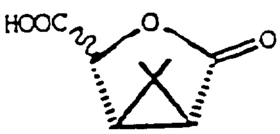

(III')

one or other form appearing as a mixture of diastereoisomers and in the form of an alkaline, alkaline-earth or magnesium salt, from which, if appropriate, the acid is isolated, then if appropriate, the diastereoisomers are isolated, then the compound of formula (III) or (III') is treated in the form of a mixture of diastereoisomers or of separate diastereoisomers or its salt, with an oxidizing agent, in order to obtain the compound of formula (I).

**2.** Process according to claim 1, characterized in that the hypohalogenite is a sodium, potassium, lithium, calcium or magnesium hypochlorite, hypobromite or hypoiodite and in particular sodium hypochlorite.

**3.** Process according to claim 1 or 2, characterized in that at least 4 equivalents of hypohalogenite are used.

**4.** Process according to any one of claims 1 to 3, characterized in that the operation takes place at a temperature between -5 and +5°C in aqueous phase.

**5.** Process according to any one of claims 1 to 4, characterized in that the operation takes place in the presence of a basic agent.

**6.** Process according to claim 5, characterized in that the basic agent is chosen from the group constituted by the alkaline and alkaline-earth hydroxides and in particular from the group constituted by sodium, potassium and calcium hydroxides.

7. Process according to claim 1, characterized in that the oxidizing agent is chosen from the group constituted by the hypohalogenous acids, the alkaline, alkaline-earth and magnesium hypohalogenites, potassium permanganate, chromic acid, periodic acid and the alkaline bismuthates and in particular a hypohalogenous acid.

8. Process according to claim 7, characterized in that the hypohalogenous acid is obtained in situ from an alkaline, alkaline-earth or magnesium hypohalogenite placed in acid medium.

9. Process according to claim 7, characterized in that the oxidizing agent is hypochlorous acid obtained in situ from sodium hypochlorite placed in acid medium.

10. Process according to claim 8 or 9, characterized in that the acid is chosen from the group constituted by the lower alkanoic acids, in particular acetic and propionic acids, and solutions of phosphates, borates and acetates.

11. Process according to claim 1, characterized in that the operation takes place without intermediate isolation of the compound of formula (III) or (III').

12. Process according to claim 11, characterized in that the oxidizing agent is as defined or as obtained in any one of claims 7 to 10.

13. Process according to claim 11 or 12, characterized in that a quantity greater than 4 equivalents of a hypohalogenite is used at the start.

14. Process according to claim 13, characterized in that a quantity greater than 4 equivalents of sodium hypochlorite is used at the start.

15. The compounds of formula (III) and (III') as defined in claim 1, as well as their alkaline, alkaline-earth or magnesium salts in the form of diastereoisomer mixtures or of separate diastereoisomers.